(19) 

**Europäisches Patentamt**
**European Patent Office**
**Office européen des brevets**

(11) **EP 4 571 594 A1**

(12) **EUROPEAN PATENT APPLICATION**

(43) Date of publication:
**18.06.2025 Bulletin 2025/25**

(51) International Patent Classification (IPC):
***G06N 5/01*** *(2023.01)* ***G06N 20/00*** *(2019.01)*

(21) Application number: **23216688.4**

(22) Date of filing: **14.12.2023**

(52) Cooperative Patent Classification (CPC):
**G06N 20/00; G06N 5/01;** G16H 30/40

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Turun Ammattikorkeakoulu Oy**
**20520 Turku (FI)**

(72) Inventors:
• **Khan, Mohammad**
**Turku (FI)**
• **Khan, Suleiman**
**Turku (FI)**
• **Kontio, Elina**
**Turku (FI)**
• **Jafaritadi, Mojtaba**
**Turku (FI)**

(74) Representative: **Moosedog Oy**
**Kurjenmäenkatu 10 B 49**
**20700 Turku (FI)**

(54) **METHOD AND SYSTEM FOR SELECTION OF COLLABORATORS IN FEDERATED LEARNING ENVIRONMENT**

(57)     Disclosed is a method (100) and a system (200) for selection of collaborators in a federated learning environment for training of a federated model (210). The method comprises initiating communication with multiple collaborators (206) providing corresponding collaborator model (208) trained on respective current dataset. The method further comprises determining a retrospective performance score for each of the multiple collaborators based on a contribution of the respective collaborator model in training of the federated model in preceding rounds. The method further comprises implementing a reinforcement learning module to select, for a current round, a subset of collaborators for utilization in training of the federated model. Herein, the subset of collaborators is selected by methodically alternating between selecting the collaborators whose respective collaborator models have not been utilized in one or more of the preceding rounds and selecting the collaborators based on the retrospective performance scores thereof.

FIG. 1

**EP 4 571 594 A1**

**Description**

TECHNICAL FIELD

The present disclosure, generally, relates to a method for selection of collaborators in a federated learning environment for training of a federated model. Moreover, the present disclosure relates to a system for selection of collaborators in a federated learning environment for training of a federated model.

BACKGROUND

Federated learning (FL) is a computational paradigm for distributed or decentralized machine learning where training data is hosted on multiple collaborators and a central server learns a consensus model by aggregating locally computed model updates. Unlike traditional centralized models, where all data is aggregated at a single location for training, federated learning enables multiple entities, referred to as 'collaborators', to train models locally on their own datasets. These locally trained models, often termed 'collaborator models', are then collectively used to update or refine a federated model (also sometimes referred to as global model). This approach ensures that raw data remains local, addressing growing concerns around data privacy, security, and governance. The decentralized nature of federated learning has found resonance across diverse sectors, from healthcare and finance to telecommunications and retail, especially in scenarios where data privacy and security are paramount.

An FL system encourages collaboration and representation, similar to systems that prioritize participation. Collaborators are chosen for training rounds based on their retrospective performance, resembling individuals competing for roles in a collaborative initiative. Furthermore, the aggregation of model updates resembles a collective consensus-building process, as collaborators contribute their insights to shape the collective model. This flexible approach accommodates differences in data, similar to variations in preferences across different entities. A cornerstone of FL's systems is balancing the needs of each collaborator with the objective of improving the model as a whole. FL facilitates collaboration and equitable participation, encourages the exchange of knowledge, and advances ML while protecting the privacy of data.

While federated learning provides data privacy and collaborative intelligence, it also introduces unique challenges not encountered in traditional centralized learning paradigms. One of the most pressing challenges is the optimal selection of collaborators for model training. Given that each collaborator possesses a unique dataset, which can vary in terms of size, distribution, quality, and relevance, the contribution of each collaborator to training of the federated model becomes a complex equation. Merely aggregating collaborator models without a strategic selection process can lead to imbalanced or biased federated models. Moreover, the dynamic and evolving nature of federated learning environments, where new collaborators can join, and data distributions can change, further exacerbates the challenge of collaborator selection. This can result in suboptimal training outcomes, reduced model accuracy, and limited generalizability of the federated model.

To address the aforementioned challenges, various heuristic-based methods for collaborator selection have been proposed in the past. Some of these methods prioritize collaborators based on the size of their datasets, under the assumption that more data equates to better training outcomes. Others focus on the diversity of data, selecting collaborators whose datasets differ significantly from the aggregated data pool, thereby aiming to enhance the model's diversity and generalizability. However, by relying heavily on simplistic metrics like dataset size or past performance, these methods may overlook potential contributors who might possess datasets of immense value, even if they are smaller in size or have not retrospectively been top performers. Another prevalent technique involves a batch-wise approach which has been the standard protocol, where available collaborators are preselected for each round of FL. However, this approach often encounters limitations in adapting to dynamic scenarios where new collaborators may join with evolving data distributions and hardware configurations.

Therefore, in light of the foregoing discussion, there exists a need to overcome the aforementioned limitations/drawbacks.

SUMMARY

The aim of the present disclosure is to provide a method and a system for dynamic and adaptive collaborator selection in federated learning, balancing between exploration of new data sources and exploitation of retrospectively effective contributors, to enhance the training outcomes of a federated model. The aim of the present disclosure is achieved by a method and a system for selection of collaborators in a federated learning environment for training of a federated model as defined in the appended independent claims to which reference is made to. Advantageous features are set out in the appended dependent claims.

Throughout the description and claims of this specification, the words "*comprise*", *"include", "have",* and *"contain"* and variations of these words, for example *"comprising"* and *"comprises"*, mean *"including but not limited to"*, and do not exclude other components, items, integers, or steps not explicitly disclosed also to be present. Moreover, the singular encompasses the plural unless the context otherwise requires. In particular, where the indefinite article is used, the specification is to be understood as contemplating plurality as well as singularity, unless the context requires otherwise.

BRIEF DESCRIPTION OF THE DRAWINGS

FIG. 1 is an illustration of a flowchart listing steps involved in a method for selection of collaborators in a federated learning environment for training of a federated model, in accordance with one or more embodiments of the present disclosure;

FIG. 2 is an illustration of a simplified schematic diagram of a system for selection of collaborators in a federated learning environment for training of a federated model, in accordance with one or more embodiments of the present disclosure; and

FIG. 3 is an illustration of various graphs depicting the model training performance on internal validation data, in accordance with an embodiment of the present disclosure.

DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practising the present disclosure are also possible.

In a first aspect, the present disclosure provides a method for selection of collaborators in a federated learning environment for training of a federated model, the method comprising:

- initiating communication with multiple collaborators, with each of the multiple collaborators providing a collaborator model trained on respective current dataset, to be utilized for training of the federated model in successive rounds;
- determining a retrospective performance score for each of the multiple collaborators, quantifying retrospective performances of the corresponding collaborator, based on a contribution of the respective collaborator model in training of the federated model in preceding rounds; and
- implementing a reinforcement learning module to select, for a current round, a subset of collaborators from the multiple collaborators to obtain the corresponding collaborator models for utilization in training of the federated model, wherein the reinforcement learning module is configured to select the subset of collaborators by methodically alternating between selecting the collaborators whose respective collaborator models have not been utilized in one or more of the preceding rounds and selecting the collaborators based on the retrospective performance scores thereof.

The present method provides a significant advancement in federated learning by synergistically combining retrospective performance scores and a reinforcement learning module for the optimized selection of collaborators. By initiating communication with multiple collaborators and harnessing their unique collaborator models, the method ensures a diverse and comprehensive foundation for the federated model. Furthermore, the consideration of retrospective performance scores provides an empirical basis for collaborator evaluation, ensuring that contributors with proven efficacy are prioritized. Moreover, the reinforcement learning module balances between utilizing the strengths of retrospectively effective collaborators and exploring the potential of underutilized ones. This methodical alternation between exploration and exploitation not only optimizes the training outcomes but also ensures equitable participation across collaborators. Together, these features provide a federated learning environment that is adaptive, inclusive, and achieves a high level of model accuracy and generalizability.

In a second aspect, the present disclosure provides a system for selection of collaborators in a federated learning environment for training of a federated model, the system comprising:

- a server; and
- a communication network to dispose the server in communication with multiple collaborators, with each of the multiple collaborators providing a collaborator model trained on respective current dataset, to be utilized for training of the federated model in successive rounds,
- wherein the server is configured to:

- determine a retrospective performance score for each of the multiple collaborators, quantifying retrospective performances of the corresponding collaborator, based on a contribution of the respective collaborator model in training of the federated model in preceding rounds; and

- implement a reinforcement learning module to select, for a current round, a subset of collaborators from the multiple collaborators to obtain the corresponding collaborator models for utilization in training of the federated model, wherein the reinforcement learning module is configured to select the subset of collaborators by methodically alternating between selecting the collaborators whose respective collaborator models have not been utilized in one or more of the preceding rounds and selecting the collaborators based on the retrospective performance scores thereof.

The system integrates the server, the communication network, and the reinforcement learning module to optimize the selection of collaborators in the federated learning environment. The capability of the central server to establish communication with diverse collaborators ensures a rich pool of data sources for training of the federated model. By methodically evaluating retrospective performance scores, the system efficiently identifies and prioritizes collaborators based on their past contributions. The reinforcement learning module further refines the selection process, balancing between retrospectively effective collaborators and those yet to be fully leveraged. This harmonious integration of features ensures that the system not only optimizes training outcomes but also promotes equitable participation, resulting in the federated model that is both robust and representative of diverse data inputs.

In the context of the present disclosure, the "federated learning environment" refers to a decentralized machine learning paradigm where multiple entities collaborate without necessarily sharing raw data, ensuring data privacy while benefiting from collective intelligence. These entities, termed as "collaborators," each possess unique datasets on which they locally train their specific models, known as "collaborator models." These collaborator models then contribute to a central or "federated model," which is a global representation synthesized from individual models without accessing the underlying data directly. The "training of the federated model" encompasses iterative processes wherein the federated model is refined and updated based on insights derived from the collaborator models across successive rounds of learning.

The method comprises initiating communication with the multiple collaborators, with each of the multiple collaborators providing the collaborator model trained on the respective current dataset, to be utilized for training of the federated model in successive rounds. Herein, the initial step involves establishing communication with multiple collaborators. This process of communication initiation facilitates the subsequent exchange of information and data necessary for the federated learning process. Each of these multiple collaborators possesses a unique model, referred to as the "collaborator model." The collaborator model from each collaborator is distinctively trained on its current dataset, a dataset that is specific to that particular collaborator and may encompass unique attributes and characteristics which may not be present in the datasets of other collaborators. As the federated learning process progresses over time, it is structured into multiple rounds. In each of these successive rounds, the collaborator models provided by the multiple collaborators are utilized in the training of the federated model. Specifically, the insights, patterns, and learnings from each collaborator model are integrated, either directly or indirectly, into the federated model, thereby refining and enhancing its performance and accuracy. This method of utilizing collaborator models in the training of the federated model ensures that the federated model benefits from the collective intelligence of all participating collaborators while adhering to the principles of data privacy and decentralization inherent to federated learning.

The method further comprises determining the retrospective performance score for each of the multiple collaborators, quantifying the retrospective performances of the corresponding collaborator, based on the contribution of the respective collaborator model in training of the federated model in preceding rounds. That is, the method involves the computation of the retrospective performance score for each of the multiple collaborators, which may be stored in a performance score repository/cache. This retrospective performance score serves as a numerical representation or metric that quantifies the past contributions and efficacy of each collaborator within the federated learning environment. To derive this score, the method involves assessing the impact and relevance of the collaborator model from each collaborator in the context of the federated model's training.

It may be appreciated that the determination of the retrospective performance score is based on a systematic evaluation of how each collaborator model influenced the training and refinement of the federated model in previous rounds of the federated learning process. Specifically, the method examines the degree to which performance or other relevant metrics of the federated model were enhanced or affected due to the inclusion of insights from a particular collaborator model. Factors that may be considered include the accuracy improvements, reduction in errors, or other enhancements in capabilities of the federated model attributable to a specific collaborator model.

By quantifying the retrospective performances of each collaborator in this manner, the method establishes a clear and empirical basis for assessing the value and significance of contributions of each collaborator. This, in turn, enables informed decision-making in subsequent rounds of federated learning, ensuring that collaborators are selected and prioritized based on a record of their past contributions and the proven efficacy of their collaborator models in the broader context of the federated learning.

The method further includes implementing the reinforcement learning module to select, for the current round, the subset of collaborators from the multiple collaborators to obtain the corresponding collaborator models for utilization in training of the federated model, wherein the reinforcement learning module is configured to select the subset of collaborators by methodically alternating between selecting the collaborators whose respective collaborator models have not been utilized in one or more of the preceding rounds and selecting the collaborators based on the retrospective performance scores thereof. This way, the method helps in determining the optimal subset of collaborators from the available multiple collaborators for a given round of federated learning. This chosen subset will provide their respective collaborator models, which, in turn, will be integrated to enhance and refine the federated model during the current training round. Herein, the reinforcement learning module acts as an advanced computational algorithm, employing a Markov decision process to make decisions based on past experiences and learnings. The reinforcement learning module operates on principles that draw parallels with human decision-making, where decisions are influenced by prior experiences and their outcomes. For the task at hand, the reinforcement learning module is specifically engineered to make selections by employing a dual strategy as discussed in the proceeding paragraphs.

On one hand, the reinforcement learning module is attuned to prioritize novelty and exploration. This is realized by its ability to identify and select those collaborators whose collaborator models have not been incorporated or utilized in one or more of the preceding rounds of federated learning. By doing so, the reinforcement learning module ensures that fresh perspectives and data points are continually introduced into the training process, thereby preventing potential biases and over-reliance on a limited set of collaborators. This aspect of operation of the reinforcement learning module caters to the ever-evolving nature of federated learning environments, where data distributions can change, and new patterns emerge over time.

Conversely, the reinforcement learning module is also designed to value consistency and proven efficacy. To this end, the reinforcement learning module considers the retrospective performance scores of the collaborators which, as previously detailed, offer a quantified representation of past contributions of each collaborator to training of the federated model. Collaborators with higher retrospective performance scores, indicating a consistent and positive impact on the federated model in prior rounds, are given preference. This ensures that the federated model continues to benefit from collaborators that have retrospectively demonstrated value.

Herein, the reinforcement learning module is configured to balance between these two strategies, i.e., exploration of new collaborators and exploitation of proven ones. By methodically alternating between these strategies, the reinforcement learning module ensures a holistic and comprehensive approach to collaborator selection, one that captures the breadth of new insights while leveraging the depth of established knowledge. This dynamic and adaptive selection process, implemented by the reinforcement learning module, positions the federated learning system for optimal training outcomes, ensuring both the robustness and generalizability of the federated model.

To realize this objective, candidate profiles of collaborators are maintained in each FL round, and collaborators are selected using reinforcement learning (RL), employing a Markov decision process. This introduces healthy competition among collaborators, ensuring effective collaboration. In the present disclosure, multi-armed bandit algorithms are applied which are well-suited and adaptable to address this challenge. Here, each collaborator can be likened to a "bandit," and the process of selecting the most promising collaborator can be framed as an optimization problem. The criteria for making this selection can be fine-tuned by striking a balance between exploration and exploitation, effectively managed through a hyperparameter. This hyperparameter can guide the training of the master model (i.e., the federated model) at the aggregator, offering the flexibility needed to optimize the selection process. One notable advantage of this approach is its ability to mitigate or eliminate system-level bias in collaborator selection during federation rounds, which is often associated with the automatic grouping of collaborators in batch-wise techniques.

In a stochastic multi-armed bandit problem, an agent must choose between K actions ("arms") in order to maximize expected cumulative rewards over time. The reward distributions corresponding to each arm are initially unknown. Multi-armed bandits balance exploring uncertain options and exploiting the currently best option.

In an embodiment, implementing the reinforcement learning module involves applying an Epsilon-Greedy algorithm configured with an exploitation rate, set between 0 and 1, for a current round, for selection of the subset of collaborators. The Epsilon-Greedy algorithm is a known algorithmic approach specifically tailored for decision-making scenarios where a balance between exploration and exploitation is required. The Epsilon-Greedy algorithm operates by utilizing a predefined parameter termed the "exploitation rate." This exploitation rate, which is a numerical value set between 0 and 1 helps in guiding the decision-making process of the algorithm during a given round of the federated learning process. Specifically, the exploitation rate defines the likelihood or probability with which the algorithm will choose to exploit the known and retrospectively proven collaborators over exploring newer or less-utilized collaborators. In a practical sense, if the exploitation rate is set closer to 1, it indicates a strong inclination towards relying on collaborators that have previously demonstrated value in the training of the federated model, as reflected by their retrospective performance scores. Conversely, if the exploitation rate is set closer to 0, it signifies a propensity to explore and integrate insights from collaborators whose models might not have been substantially utilized in preceding rounds.

In an embodiment, the method further comprises generating a random number set between 0 and 1, for the current round, wherein the Epsilon-Greedy algorithm is configured to:

- select the subset of collaborators from the multiple collaborators with higher of the corresponding retrospective performance scores compared to rest of collaborators from the multiple collaborators when the random number is below the exploitation rate, and
- select the subset of collaborators from the multiple collaborators with lower of the corresponding retrospective performance scores compared to rest of collaborators from the multiple collaborators when the random number is above the exploitation rate.

That is, for the selection of the subset of collaborators in a current round, the Epsilon-Greedy algorithm commences by generating a random number, typically between 0 and 1. This generated number is then compared against the predefined exploitation rate. If the random number is below the exploitation rate, the algorithm interprets this as "exploitation round," leading to the selection of collaborators based on their established retrospective performance scores. However, if the random number exceeds the exploitation rate, the algorithm this as "exploration round," prompting the selection of collaborators that explore potentially undiscovered value to the federated learning process.

In particular, using the epsilon-greedy algorithm, the agent explores arms randomly with a probability of $\varepsilon$, and exploits the best-known arm with a probability of $1-\varepsilon$. This is done to balance between exploration (finding better arms) and exploitation (gaining rewards from known good arms).

Let $Q(a)$ be the estimated value of arm $a$, and $N(a)$ be the number of times arm $a$ has been pulled. The algorithm can be described as follows:

1. Initialize $Q(a)$ and $N(a)$ for all arms.

2. Repeat for each trial:

- With probability $\varepsilon$, select a random arm

$$a = \text{rand\_int}\,(1, K),$$

where $K$ is the number of arms.
- Otherwise, select the arm with the highest estimated value:

$$a = \text{argmax}_a\, Q(a).$$

- Pull arm a, receive reward $R$, and update estimates:

$$N(a) \leftarrow N(a) + 1$$

$$Q(a) \leftarrow Q(a) + N(a)1 \cdot (R - Q(a))$$

The incorporation of the Epsilon-Greedy algorithm within the reinforcement learning module enables a systematic approach to collaborator selection. By using the exploitation rate as a parameter, the method ensures adaptability, allowing the federated learning process to adjust between leveraging proven collaborators and discovering potential new collaborators. This dynamic equilibrium, facilitated by the Epsilon-Greedy algorithm, assures that training of the federated model remains comprehensive, adaptive, and is continually enhanced.

In an embodiment, implementing the reinforcement learning module involves applying an Upper Confidence Bounds (UCB) algorithm, configured to use an average retrospective performance score calculated based on an average of the retrospective performance scores of the multiple collaborators across the preceding rounds, for selection of the subset of collaborators. The UCB algorithm is also a known strategy in decision-making processes, especially where decisions are influenced by uncertainties associated with potential rewards. The UCB algorithm involves balancing the act of exploiting actions with known rewards and exploring actions whose rewards are uncertain but might be potentially higher.

For the task of selecting a subset of collaborators within the federated learning environment, the UCB algorithm is configured to utilize the average retrospective performance score, which is derived by computing the mean of the retrospective performance scores pertaining to all the multiple collaborators. These individual retrospective performance scores, which have been previously discussed, provide insights into each collaborator's past contributions and efficacy in training of the federated model across previous rounds. By aggregating these scores, the UCB algorithm provides a consolidated metric that represents the collective performance of all collaborators over time.

It may be understood that when the UCB algorithm is utilized for the selection process in a given round, it leverages the average retrospective performance score in conjunction with individual retrospective performance scores to make informed decisions. Specifically, the algorithm aims to maximize a combined score for each collaborator, which is typically the sum of their retrospective performance score and a bonus term. This bonus term often encapsulates the uncertainty or potential upside of choosing a particular collaborator. It ensures that collaborators that may not have been frequently selected in the past, but show potential, are not overlooked.

In an embodiment, the method further comprises computing an absolute distance of the retrospective performance score of each one of the multiple collaborators and the average of the retrospective performance scores, wherein the UCB algorithm is configured to:

- select the subset of collaborators from the multiple collaborators with higher of the corresponding absolute distances compared to rest of collaborators from the multiple collaborators in one of two successive rounds, and
- select the subset of collaborators from the multiple collaborators with lower of the corresponding absolute distances compared to rest of collaborators from the multiple collaborators in other of two successive rounds.

Herein, the absolute distance represents the magnitude of deviation or difference of an individual collaborator's retrospective performance from the collective average, irrespective of the direction of this deviation. By calculating this absolute distance, the method provides a quantifiable metric that includes the relative standing of each collaborator in comparison to the overall performance landscape. Specifically, the UCB algorithm employs an alternating strategy across successive rounds of the federated learning process to use the computed absolute distances for the selection process.

In one of the two successive rounds, the UCB algorithm gives preference to those collaborators whose corresponding absolute distances are higher compared to the rest of the collaborators from the multiple collaborators. This means that in this particular round, collaborators that exhibit a significant deviation (be it positive or negative) from the collective average are prioritized. In other of the two successive rounds, the UCB algorithm prefers those collaborators whose corresponding absolute distances are lower compared to the rest. This allows the algorithm to identify collaborators whose retrospective performances are closer to the collective average.

In particular, the UCB algorithm balances exploration and exploitation by choosing arms with the potential to have high rewards based on their upper confidence bounds. This ensures that arms that have been pulled less are given a chance to be explored more. Herein, let t be the current trial, and C be a parameter that controls the exploration-exploitation trade-off. The UCB algorithm can be outlined as follows:

1. Initialize Q(a) and N(a) for all arms.

2. Repeat for each trial:

- Select the arm that maximizes the upper confidence bound:

$$a = \arg\max{}_a \left( Q(a) + C \cdot \sqrt{\frac{\ln(t)}{N(a)}} \right)$$

- Pull arm *a*, receive reward *R*, and update estimates *N(a)* and *Q(a)* as in the epsilon-greedy strategy.

In the UCB equation, the term $\sqrt{\frac{\ln(t)}{N(a)}}$ represents the exploration bonus, which decreases with the number of times arm *a* has been pulled *N(a)* and increases with the number of trials (t).

Thereby, the UCB algorithm ensures a systematic and balanced integration of collaborators over successive rounds. By methodically alternating between collaborators with high and low absolute distances, the UCB algorithm provides a holistic training environment where both exceptional performances and consistent, average-aligned performances are utilized. Thus, the integration of the UCB algorithm within the reinforcement learning module provides a data-driven approach to collaborator selection. This ensures that the federated learning process remains dynamic and comprehensive, optimizing the training outcomes of the federated model.

In an embodiment, a number of collaborators in the set of collaborators is fixed as a percentage of a number of multiple collaborators. That is, the method adopts a dynamic approach to collaborator selection by specifying that the set of collaborators should represent a certain proportion of the entire pool of multiple collaborators. This proportion, determined as a fixed percentage, can be fine-tuned to accommodate different scenarios and requirements. This design choice aligns with the ever-evolving nature of federated learning environments, where the number of collaborators may fluctuate over time. By fixing the set of collaborators as a percentage of the total collaborators, the method effectively adjusts to variations in the pool of available collaborators. In scenarios where new collaborators join the federated learning environment, the fixed percentage ensures their integration. Simultaneously, if certain collaborators become inactive or exit the environment, the method accommodates these changes by recalibrating the set of collaborators as a percentage of the updated total.

In an embodiment, the method further comprises maintaining, for each one of preceding rounds, a record of collaborators whose collaborator models have been utilized for training of the federated model therein, wherein the reinforcement learning module is configured to select the subset of collaborators by methodically alternating between selecting collaborators based on the maintained record and the retrospective performance scores thereof. That is, for each of the preceding rounds, the method keeps a record that identifies and tracks the collaborators whose collaborator models have participated in the training of the federated model. This retrospective record acts as a repository of past engagement, recording the collaborators' recent contributions to the federated learning. By maintaining this retrospective record, the proposed approach is able to provide insights into the recent participation patterns and contributions of individual collaborators.

In particular, the reinforcement learning module leverages this retrospective record in conjunction with the collaborators' retrospective performance scores to guide its selection process. The configuration of the reinforcement learning module is characterized by a methodical alternation between two distinct criteria for selecting the subset of collaborators, optimizing the federated learning process. In one phase of this alternation, the reinforcement learning module refers to the maintained record, and systematically considers collaborators whose models have been actively utilized in one or more of the preceding rounds. This criterion values and reinforces the continued involvement of collaborators who have demonstrated consistent contributions, thereby maintaining a degree of stability and reliability in the training process. In the complementary phase of the alternation, the reinforcement learning module considers the retrospective performance scores of collaborators, and utilizes these scores as a metric to evaluate and select collaborators whose retrospective performance suggests potential value, irrespective of their past involvement. This criterion introduces an element of exploration, resulting in the inclusion of collaborators whose performance may have been underutilized but has the potential to yield valuable insights.

In the present federated segmentation framework, specifically, for the Epsilon-Greedy algorithm, the log maintains the validation performance history of all collaborators across rounds. The Epsilon-Greedy algorithm is applied for collaborator selection, with a fixed $\varepsilon = 0.8$ balancing exploration and exploitation. Specifically, a random number is generated each round. If this random number is below the exploitation rate of 0.2, the top 20% highest performing collaborators from the logs are selected to optimize model performance. But if this random number is above 0.2, the bottom 20% lowest performing collaborators are chosen to encourage exploration. This alternating selection approach allows both leveraging the strengths of high-performing collaborators and discovering the benefits of underutilized collaborators over the federated learning lifecycle. Like epsilon-greedy bandit algorithms, the federated framework exploits known rewards while still exploring uncertain options, harmonizing the collective learning process.

Below are steps involved in implementation of the Epsilon-Greedy algorithm for collaborator selection.

**Input:**
- *log:* A list containing the aggregate validation performance of collaborators from previous rounds.
- *exploitation_rate*: The desired exploitation rate, e.g., 0.2 for 20% exploitation.

**Procedure:**
- *selected_collaborators*: A list of collaborator indices selected for the next round.

1. Initialize *num_collaborators* as the length of log.
2. Initialize *num_to_select* as the integer value of *num_collaborators* x 0.2 (equivalent to 20% of collaborators).
3. Generate a random number *random_num* between 0 and 1 to determine the exploration rate.
4. **if** *random_num* < *exploitation_rate* **then**
5. Sort collaborator indices in decreasing order of their performance in *log.*

(continued)

6. Set *selected_collaborators* as the first *num_to_select* indices from the sorted list.

7. **else**

8. Sort collaborator indices in increasing order of their performance in log.

9. Set *selected_collaborators* as the first *num_to_select* indices from the sorted list.

**Output:**

10. **return** *selected_collaborators* as the final list of collaborator indices for the next federation round.

This collaborator election approach is well-suited for real-world scenarios as it accommodates scenarios where new collaborators join the federation, and the data distribution undergoes constant changes due to the inclusion of new patient data within a collaborator's domain. The Epsilon-Greedy approach not only ensures equitable participation of all collaborators but also contributes to the optimization of generalization capabilities of the federated model.

On the other hand, in the UCB approach for collaborator selection, a log stores the validation performance history of all collaborators across federation rounds, including the average validation score. The UCB score for each collaborator is calculated as the observed mean reward (validation score) of all available collaborators and an exploration bonus proportional to the standard deviation of their observed rewards. This exploration bonus encapsulates uncertainty about collaborators. Distinct selection strategies are employed in alternating rounds. In even rounds, the top 20% of collaborators by highest UCB score are chosen to maximize expected performance. In odd rounds, the bottom 20% are selected to explore uncertain collaborators. The selected collaborators then participate in training the federated model during the subsequent round. This alternating approach balances leveraging highly rewarding collaborators with exploring uncertain options, providing a principled way to optimize the collective learning process. Over time as uncertainty about collaborator distributions decreases, larger UCB exploration bonuses help identify new high-performing candidates as the federated system evolves.

Below are steps involved in implementation of the UCB algorithm for collaborator selection.

**Input:**

• *log*: A list containing the aggregate validation performance of collaborators from previous rounds.

**Procedure:**

• *selected collaborators: A list of collaborator indices selected for the next round.*

1. Initialize *round_number* as the current round number.

2. Calculate the average validation score, denoted as *avg_score*.

3. **for** each collaborator *i* **do**

4. Calculate the absolute distance of individual collaborator validation score $s_i$ from the average validation score:

5. $d_i = |s_i - avg\_score|$

6. **if** *round_number* is even **then**

7. Sort collaborator indices based on the absolute distance scores $d_i$ in increasing order.

8. **else**

9. Sort collaborator indices based on the absolute distance scores $d_i$ in decreasing order.

10. Initialize *num_collaborators* as the length of *log*.

11. Initialize num_to_select as the integer value of num_collaborators 0.2 (equivalent to 20%).

12. Set *selected_collaborators* as the first *num_to_select* indices from the sorted list.

**Output:**

13. return *selected_collaborators* as the final list of collaborator indices for the next federation round.

Thus, the present method ensures a systematic and balanced integration of collaborators over successive rounds. By methodically alternating between collaborators, the proposed algorithms foster a holistic training environment where both exceptional performances and consistent, average-aligned performances are valued and utilized. Such a strategy not only enriches the federated learning process but also ensures that the federated model remains adaptive, comprehensive, and attuned to the diverse insights offered by the multiple collaborators.

In one or more embodiments, the federated learning environment is implemented for semantic segmentation of medical image data. As used herein, "semantic segmentation" refers to the process of partitioning or segmenting medical images into distinct regions or categories based on the semantic meaning of the content. In the medical domain, this typically involves the precise delineation of structures, abnormalities, or specific features within medical images. Semantic segmentation is implemented for tasks such as organ delineation, tumour localization, and anomaly detection, enabling clinicians to make informed decisions based on the segmented regions. In this environment, multiple collaborators, often representing diverse healthcare institutions, research facilities, or medical imaging centres, collectively contribute their expertise and data resources to train the federated model, which, in turn, is implemented for semantic segmentation of medical image data.

In one or more embodiments, the respective current dataset corresponding to the collaborator model from each of the multiple collaborators, as implemented in training of the federated model for the semantic segmentation of the medical image data, comprises at least one of magnetic resonance imaging (MRI) data, positron emission tomography (PET) data, single photon positron emission tomography (SPECT) data, computed tomography (CT) data, X-ray image data, microscopic image data. Herein, MRI is a non-invasive imaging modality that provides detailed anatomical and functional information, and is commonly used for imaging soft tissues, such as the brain, muscles, and organs. PET imaging involves the use of radiotracers to visualize metabolic processes in the body, and is valuable for detecting diseases, including cancer, and assessing organ function. SPECT is another nuclear medicine imaging technique used to examine the distribution of radiotracers within the body, and aids in diagnosing various medical conditions. CT scans use X-rays to create detailed cross-sectional images of the body, and are widely used for diagnosing injuries, tumours, and other medical conditions. X-ray imaging is a standard method for visualizing bones and detecting fractures, dental issues, and pulmonary conditions. Microscopic imaging involves the examination of tissues and cells at a microscopic level and is used for pathology and research applications.

As discussed, each collaborator participating in the federated learning environment contributes the current dataset that aligns with the requirements of the collaborative task. These datasets, curated and maintained by the individual collaborators, form the basis for training the collaborator models and subsequently, the federated model. The inclusion of these diverse imaging modalities allows the federated model to learn from a varied set of data sources, enhancing its ability to perform semantic segmentation on medical image data across different modalities. This breadth of data sources empowers the model to generalize its learnings and effectively address a wide range of clinical scenarios, ultimately benefiting medical professionals and improving patient care.

The present disclosure also relates to the system for selection of collaborators in the federated learning environment for training of the federated model as described above. Various embodiments and variants disclosed above, with respect to the aforementioned method, apply *mutatis mutandis* to the system.

Herein, the server is adept at establishing communication with the multiple collaborators via the communication network. The server serves as the central hub, orchestrating the collaborative efforts within the federated learning environment. This ensures that a diverse array of collaborator models, each trained on distinct datasets, can be utilized to enrich training of the federated model. The capability of the server to compute retrospective performance scores for each collaborator serves as a quantitative metric, capturing the collaborator's past effectiveness and impact on the collective learning process. The reinforcement learning module, as implemented by the server, is designed to make informed decisions regarding collaborator selection for the current training round. Its primary objective is to strategically choose a subset of collaborators from the larger pool of multiple collaborators. These selected collaborators are actively utilized in the training of the federated model. In some implementations, the server may be equipped with homomorphic encryption allowing computations on private and encrypted data through a multi-party computation protocol.

In an embodiment of the system, the server is configured to implement the reinforcement learning module by applying an Epsilon-Greedy algorithm configured with an exploitation rate, set between 0 and 1, for a current round, for selection of the subset of collaborators.

In an embodiment of the system, the server is further configured to generate a random number set between 0 and 1, for the current round, and wherein the Epsilon-Greedy algorithm is configured to:

-    select the subset of collaborators from the multiple collaborators with higher of the corresponding retrospective performance scores compared to rest of collaborators from the multiple collaborators when the random number is below the exploitation rate, and

-    select the subset of collaborators from the multiple collaborators with lower of the corresponding retrospective performance scores compared to rest of collaborators from the multiple collaborators when the random number is above the exploitation rate.

In an embodiment of the system, the server is further configured to implement the reinforcement learning module by applying an Upper Confidence Bounds (UCB) algorithm, configured to use an average retrospective performance score calculated based on an average of the retrospective performance scores of the multiple collaborators across the preceding rounds, for selection of the subset of collaborators.

In an embodiment of the system, the server is further configured to compute an absolute distance of the retrospective performance score of each one of the multiple collaborators and the average of the retrospective performance scores, and wherein the UCB algorithm is configured to:

- select the subset of collaborators from the multiple collaborators with higher of the corresponding absolute distances compared to rest of collaborators from the multiple collaborators in one of two successive rounds, and

- select the subset of collaborators from the multiple collaborators with lower of the corresponding absolute distances compared to rest of collaborators from the multiple collaborators in other of two successive rounds.

The present disclosure further relates to a computer program comprising computer executable program code, when executed the program code controls a computer to perform the method as described in the preceding paragraphs.

EXPERIMENTAL PART

The experimental framework was rigorously formulated to optimize the process of federated lesion segmentation, encompassing various methodological components. These components include streamlined aggregation methodologies, judicious selection of client participants, round-specific training protocols, and efficient strategies for communication. The overarching objective was to achieve an optimal selection of collaborating participants by means of reinforcement learning (RL) strategies while ensuring the proficient aggregation of model updates obtained from specific contributors.

The current study utilized real-world multi-parametric magnetic resonance imaging (mpMRI) data from glioblastoma (GBM) cases made publicly available through the Federated Tumour Segmentation (FeTS) 2022 challenge. GBM is a clinically aggressive brain malignancy classified as a grade IV astrocytoma. The dataset encompassed 1251 mpMRI scans from confirmed GBM patients, including native T1-weighted images, post-gadolinium contrast enhanced T1-weighted images, T2-weighted images, and T2 Fluid Attenuated Inversion Recovery (FLAIR) images. These cases were derived from the Brain Tumour Segmentation (BraTS) Continuous Challenge dataset, which aims to promote the development and benchmarking of state-of-the-art algorithms for consistent brain tumour delineation across institutions. Specifically, the GBM cohort examined in this study represented a subset of diffuse glioma cases from BraTS containing ground truth segmentations of tumour sub-regions (edema, enhancing core, and necrotic core). By utilizing real-world training data from FeTS and BraTS, it was possible to evaluate federated learning for robust GBM segmentation under controlled conditions with known ground truth labels. An additional set consisting of 219 patients was reserved to serve as a validation cohort. The collaborative aspect of this study involved the active participation of 33 institutions who played a pivotal role in the partitioning of the dataset.

The mpMRI data utilized in this study was sourced from multiple academic institutions and pre-processed according to standard protocols including rigid registration, brain extraction, alignment, resolution resampling to uniform voxel sizes, and skull stripping for isolation of intracranial content. To develop and evaluate a federated learning approach for collaborative training of a segmentation model without sharing patient data, Intel's Federated Learning OpenFL framework along with a U-Net convolutional neural network (CNN) architecture was implemented. The federated learning methodology employed a randomly selected subset of participating sites to train local models and update the global model in each round approximately 20% of total sites actively contributed to the global model training in a given round. This ensured that no one site had perpetual control over model updates, promoting decentralized collaboration. The evaluation criteria encompassed DICE similarity and Hausdorff (95%) distance, providing a robust framework for the assessment of the efficacy of the aggregation rounds.

This experimental configuration served as a robust foundation for the systematic evaluation of performance enhancements resulting from the proposed RL-based collaborator selection and model aggregation strategies within the overarching framework of FL. The hyperparameters used are shown in Table I.

Table I: HYPERPARAMETERS USED IN AGGREGATION ALGORITHMS.

| Hyperparameter | SimHAgg |
| --- | --- |
| Learning rate | 5e-5 |
| Epochs per round | 1.0 |
| Communication rounds | 20 |

A brief summary of the collaborator selection process utilizing reinforcement techniques with the adaptation of modified similarity weighted aggregation (RL-SimHAgg) is provided. The results emphasize that the proposed methods exhibit swift convergence and sustained stability as the learning process progresses, encompassing all the assessed criteria.

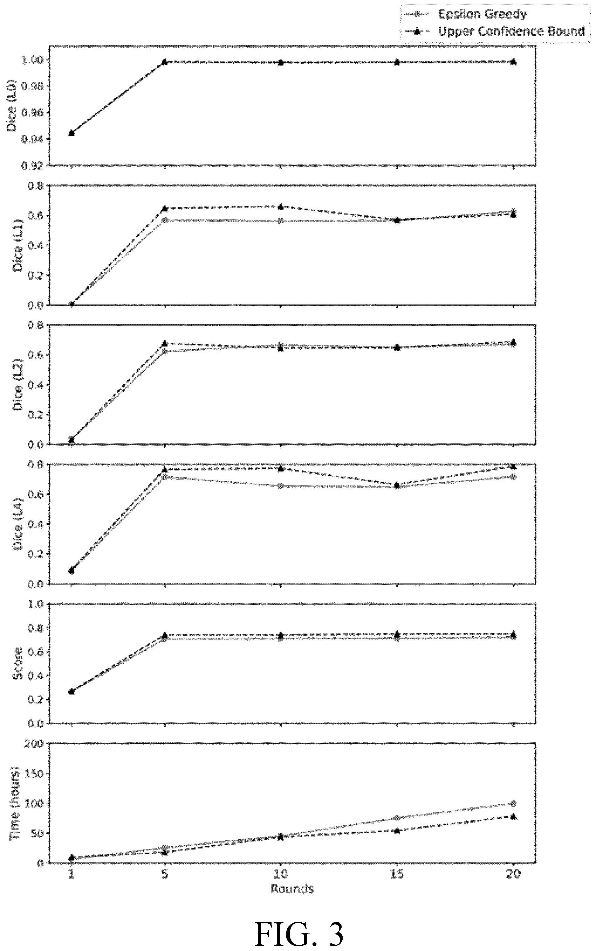

_Model training and performance using internal validation data:_ FIG. 3 shows the model training performance on internal validation data observed over a duration of 7.5 days of computational time dedicated to model training. The labels utilized in FIG. 3, namely 0, 1, 2, and 4, serve to delineate distinct classes relevant to brain tumour segmentation. Specifically, label 0 signifies normal brain tissue, label 1 represents the entire tumour along with the surrounding edematous area, label 2 corresponds to the tumour core, and label 4 is used to denote the enhancing region of the tumour.

_Model performance using external validation data:_ The results also assess the performance of the proposed approach using 219 instances of external validation data, as detailed in Table II below. In general, the Epsilon-Greedy algorithm exhibits superior performance in the segmentation of the tumour core, whereas the UCB algorithm demonstrates improved performance in the segmentation of the enhancing tumour region. The difference between the two approaches did not seem to be notable in terms of Dice score. However, the UCB approach resulted in a smaller Hausdorff (95%) as compared to the Epsilon-Greedy approach for all tumour regions after 20 training rounds. The UCB approach showed faster and higher convergence scores as well.

Table II: PERFORMANCE ON VALIDATION DATA

| Metrics | EG Approach | UCB Approach |
|---|---|---|
| Dice ET | 0.6981 | 0.7094 |
| Dice TC | 0.7331 | 0.7151 |
| Dice WT | 0.8067 | 0.8007 |
| Hausdorff (95%) ET | 40.928 | 30.424 |
| Hausdorff (95%) TC | 28.875 | 25.917 |
| Hausdorff (95%) WT | 32.242 | 23.140 |
| Sensitivity ET | 0.6654 | 0.6712 |
| Sensitivity TC | 0.7069 | 0.6809 |
| Sensitivity WT | 0.8395 | 0.8063 |
| Specificity ET | 0.9998 | 0.9998 |
| Specificity TC | 0.9997 | 0.9998 |
| Specificity WT | 0.9984 | 0.9988 |

This work highlights the efficacy of incorporating RL strategies into the collaborator selection process. By harnessing the principles of Epsilon-Greedy and UCB algorithms, the proposed techniques have addressed the critical challenge of optimally selecting collaborators from a diverse pool of available collaborators for each round of FL.

The findings demonstrate that the integration of RL algorithms enables the system to dynamically adapt and make intelligent decisions in real-time, leading to a more refined and efficient training process for the machine learning model. Currently, there is a limitation that data distributions and the number of available collaborators are fixed. Moreover, the research illustrates the significance of considering the inherent heterogeneity in collaborators' contributions and data distributions, as reflected in non-IID data scenarios. By adopting multi-armed bandit-like strategies, a balance is achieved between exploration and exploitation, allowing for the identification and selection of optimal collaborators based on their performance histories. This approach cultivates an electoral environment wherein collaborators compete to participate in subsequent federation rounds, fostering healthy competition and enhancing the overall quality of the trained model.

In the current federated lesion segmentation experiments, two limitations are that the number of collaborators is fixed and the data distribution across collaborators remains static over rounds. To deal with this simplified scenario, an alternating selection strategy is employed that chooses the best and worst collaborators in even and odd rounds respectively. However, in a dynamic real-world setting, both the number of collaborators and the data distribution at each collaborator would vary over time. To adapt this selection strategy for such evolving conditions, the proposed techniques employ more advanced multi-armed bandit algorithms that dynamically select collaborators to maximize the overall expected reward or utility in each round.

Specifically, bandit algorithms such as Thompson sampling or upper confidence bound algorithms could be applied to balance exploring new collaborators versus exploiting known high performers. The expected reward for collaborator selection can be defined using metrics such as model accuracy, data diversity, or other factors correlated with improved global model performance. Environment conditions like changing collaborator populations and data drift can be encoded in the prior selection for Bayesian approaches like Thompson sampling. This adaptive online optimization of collaborator selection based on changing utility and environment conditions can maximize overall learning performance in dynamic federated networks compared to fixed alternating selection rules. More research is still needed to develop such responsive selection algorithms for real-world FL.

The findings presented herein underscore the potential of RL algorithms in enhancing the FL process. The present disclosure further envision the exploration of advanced RL paradigms and the investigation of their application in diverse domains to further refine the collaborator selection process. Alternative RL methodologies have been explored to effectively optimize collaborator selection for FL model training. These methodologies involve an algorithm that assesses the states of collaborators within the FL system and defines an action space to navigate to subsequent system states. In this context, a neural network, referred to as the Q-network, operates within the framework of Deep Q-Network (DQN) to estimate Q-values associated with a range of state-action pairs. These Q-values encapsulate the projected cumulative rewards achievable by taking an action from a given state. Throughout the training process, the DQN strives to minimize disparities between predicted Q-values and actual rewards earned. The transition between states is governed by the policy design, encompassing exploration strategies and recognized rewards. This state-to-state progression adheres to the guidance provided by the Bellman equation, a fundamental concept in RL that articulates the relationship between a state's value and the anticipated cumulative reward that can be obtained from that state onward. This methodology effectively balances immediate rewards with forecasts of forthcoming rewards. With the proliferation of FL across various industries, the proposed approach holds promise in bolstering the efficiency, robustness, and scalability of this decentralized learning paradigm.

The method and the system of present disclosure have strong commercial potential in the field of privacy, artificial intelligence and secure use and processing of health data. The use of reinforcement learning and multi-armed bandit algorithms to enhance collaborator selection addresses a critical challenge in federated learning. By improving the accuracy and reliability of deep learning models for medical image segmentation tasks, the present disclosure can have significant applications in healthcare institutions, medical research organizations, and companies involved in medical imaging technologies. Potential users of the present disclosure include, but not limited to, radiologists, healthcare providers, medical device manufacturers, and AI development companies focusing on medical applications. The ability to select the most suitable collaborators in a dynamic environment can lead to better segmentation outcomes, improved diagnostic accuracy, and more efficient use of resources, making it an attractive solution for commercial adoption.

DETAILED DESCRIPTION OF THE DRAWINGS

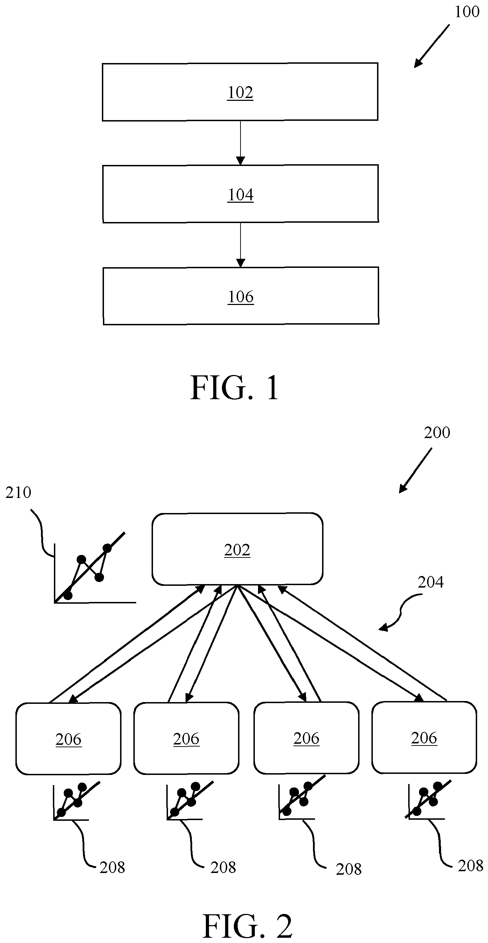

Referring to FIG. 1, illustrated is a flowchart listing steps 102-106 involved in a method 100 for selection of collaborators in a federated learning environment for training of a federated model, in accordance with an embodiment of the present disclosure. At step 102, the method 100 includes initiating communication with multiple collaborators, with each of the multiple collaborators providing a collaborator model trained on respective current dataset, to be utilized for training of the federated model in successive rounds. At step 104, the method 100 includes determining a retrospective performance score for each of the multiple collaborators, quantifying retrospective performances of the corresponding collaborator, based on a contribution of the respective collaborator model in training of the federated model in preceding rounds. At step 106, the method 100 includes implementing a reinforcement learning module to select, for a current round, a subset of collaborators from the multiple collaborators to obtain the corresponding collaborator models for utilization in training of the federated model, wherein the reinforcement learning module is configured to select the subset of collaborators by methodically alternating between selecting the collaborators whose respective collaborator models have not been utilized in one or more of the preceding rounds and selecting the collaborators based on the retrospective performance scores thereof.

It may be appreciated that the above steps are only illustrative and other alternatives can also be provided where one or more steps are added, one or more steps are removed, or one or more steps are provided in a different sequence without departing from the spirit and the scope of the present disclosure.

Referring to FIG. 2, illustrated is a simplified schematic diagram illustration of a system 200 for selection of collaborators in a federated learning environment for training of a federated model 210, in accordance with an embodiment of the present disclosure. As shown, the system 200 comprises a server 202, and a communication network 204 to dispose the server 202 in communication with each of the multiple collaborators 206. Herein, each of the multiple collaborators 206 provides a collaborator model 208 trained on respective current dataset, to be utilized for training of the federated model 210 in successive rounds. The server 202 is configured to determine a retrospective performance score for each of the multiple collaborators 206, quantifying retrospective performances of the corresponding collaborator, based on a contribution of the respective collaborator model 208 in training of the federated model 210 in preceding rounds. The server 202 is further configured to implement a reinforcement learning module to select, for a current round, a subset of collaborators from the multiple collaborators 206 to obtain the corresponding collaborator models for utilization in training of the federated model 210. Herein, the reinforcement learning module is configured to select the subset of collaborators by methodically alternating between selecting the collaborators whose respective collaborator models have not been utilized in one or more of the preceding rounds and selecting the collaborators based on the retrospective performance scores thereof.

Referring to FIG. 3, illustrated are various graphs depicting the model training performance on internal validation data, in accordance with an embodiment of the present disclosure. Herein, the horizontal axis refers to the number of rounds and the vertical axis to the performance metrics. Further, 'L0' represents Label 0, 'L1' represents Label 1, 'L2' represents Label 2, 'L4' represents Label 4, 'Score' represents Projected Convergence Score, and 'Time' represents Simulation Time (Hours).

Modifications to embodiments of the present disclosure described in the foregoing are possible without departing from the scope of the present disclosure as defined by the accompanying claims. Expressions such as "including", "comprising", "incorporating", "have", "is" used to describe and claim the present disclosure are intended to be construed in a non-exclusive manner, namely allowing for items, components or elements not explicitly described also to be present. Reference to the singular is also to be construed to relate to the plural.

## Claims

1. A method (100) for selection of collaborators in a federated learning environment for training of a federated model (210), the method (100) comprising:

   - initiating communication with multiple collaborators (206), with each of the multiple collaborators (206) providing a collaborator model (208) trained on respective current dataset, to be utilized for training of the federated model (210) in successive rounds;
   - determining a retrospective performance score for each of the multiple collaborators (206), quantifying retrospective performances of the corresponding collaborator, based on a contribution of the respective collaborator model (208) in training of the federated model (210) in preceding rounds; and
   - implementing a reinforcement learning module to select, for a current round, a subset of collaborators from the multiple collaborators (206) to obtain the corresponding collaborator models (208) for utilization in training of the federated model (210), wherein the reinforcement learning module is configured to select the subset of collaborators by methodically alternating between selecting the collaborators whose respective collaborator models (208) have not been utilized in one or more of the preceding rounds and selecting the collaborators based on the retrospective performance scores thereof.

2. A method (100) according to claim 1, wherein implementing the reinforcement learning module involves applying an Epsilon-Greedy algorithm configured with an exploitation rate, set between 0 and 1, for a current round, for selection of the subset of collaborators.

3. A method (100) according to claim 2 further comprising generating a random number set between 0 and 1, for the current round, wherein the Epsilon-Greedy algorithm is configured to:

   - select the subset of collaborators from the multiple collaborators (206) with higher of the corresponding retrospective performance scores compared to rest of collaborators from the multiple collaborators (206) when the random number is below the exploitation rate, and
   - select the subset of collaborators from the multiple collaborators (206) with lower of the corresponding retrospective performance scores compared to rest of collaborators from the multiple collaborators (206) when the random number is above the exploitation rate.

4. A method (100) according to any of claims 1-3, wherein implementing the reinforcement learning module involves applying an Upper Confidence Bounds (UCB) algorithm, configured to use an average retrospective performance score calculated based on an average of the retrospective performance scores of the multiple collaborators (206) across the preceding rounds, for selection of the subset of collaborators.

5. A method (100) according to claim 4 further comprising computing an absolute distance of the retrospective performance score of each one of the multiple collaborators (206) and the average of the retrospective performance scores, wherein the UCB algorithm is configured to:

   - select the subset of collaborators from the multiple collaborators (206) with higher of the corresponding absolute distances compared to rest of collaborators from the multiple collaborators (206) in one of two successive rounds, and
   - select the subset of collaborators from the multiple collaborators (206) with lower of the corresponding absolute distances compared to rest of collaborators from the multiple collaborators (206) in other of two successive rounds.

6. A method (100) according to any of preceding claims, wherein a number of collaborators in the set of collaborators is fixed as a percentage of a number of multiple collaborators (206).

7. A method (100) according to any of preceding claims further comprising maintaining, for each one of preceding rounds, a record of collaborators whose collaborator models (208) have been utilized for training of the federated model (210) therein, wherein the reinforcement learning module is configured to select the subset of collaborators by methodically alternating between selecting collaborators based on the maintained record and the retrospective performance scores thereof.

8. A method (100) according to any of preceding claims, wherein the federated learning environment is implemented for semantic segmentation of medical image data.

9. A method (100) according to claim 8, wherein the respective current dataset corresponding to the collaborator model (208) from each of the multiple collaborators (206), as implemented in training of the federated model (210) for the semantic segmentation of the medical image data, comprises at least one of magnetic resonance imaging (MRI) data, positron emission tomography (PET) data, single photon positron emission tomography (SPECT) data, computed tomography (CT) data, X-ray image data, microscopic image data.

10. A system (200) for selection of collaborators in a federated learning environment for training of a federated model (210), the system (200) comprising:

   - a server (202); and
   - a communication network (204) to dispose the server (202) in communication with multiple collaborators (206), with each of the multiple collaborators (206) providing a collaborator model (208) trained on respective current dataset, to be utilized for training of the federated model (210) in successive rounds,
   - wherein the server (202) is configured to:

      - determine a retrospective performance score for each of the multiple collaborators (206), quantifying retrospective performances of the corresponding collaborator, based on a contribution of the respective collaborator model (208) in training of the federated model (210) in preceding rounds; and
      - implement a reinforcement learning module to select, for a current round, a subset of collaborators from the multiple collaborators (206) to obtain the corresponding collaborator models (208) for utilization in training of the federated model (210), wherein the reinforcement learning module is configured to select the subset of collaborators by methodically alternating between selecting the collaborators whose respective collaborator models (208) have not been utilized in one or more of the preceding rounds and selecting the collaborators based on the retrospective performance scores thereof.

11. A system (200) according to claim 10, wherein the server (202) is configured to implement the reinforcement learning module by applying an Epsilon-Greedy algorithm configured with an exploitation rate, set between 0 and 1, for a current round, for selection of the subset of collaborators.

12. A system (200) according to claim 11, wherein the server (202) is further configured to generate a random number set between 0 and 1, for the current round, and wherein the Epsilon-Greedy algorithm is configured to:

   - select the subset of collaborators from the multiple collaborators (206) with higher of the corresponding retrospective performance scores compared to rest of collaborators from the multiple collaborators (206) when the random number is below the exploitation rate, and
   - select the subset of collaborators from the multiple collaborators (206) with lower of the corresponding retrospective performance scores compared to rest of collaborators from the multiple collaborators (206) when the random number is above the exploitation rate.

13. A system (200) according to any of claims 10-12, wherein the server (202) is further configured to implement the reinforcement learning module by applying an Upper Confidence Bounds (UCB) algorithm, configured to use an average retrospective performance score calculated based on an average of the retrospective performance scores of the multiple collaborators (206) across the preceding rounds, for selection of the subset of collaborators.

**14.** A system (200) according to claim 13, wherein the server (202) is further configured to compute an absolute distance of the retrospective performance score of each one of the multiple collaborators (206) and the average of the retrospective performance scores, and wherein the UCB algorithm is configured to:

- select the subset of collaborators from the multiple collaborators (206) with higher of the corresponding absolute distances compared to rest of collaborators from the multiple collaborators (206) in one of two successive rounds, and
- select the subset of collaborators from the multiple collaborators (206) with lower of the corresponding absolute distances compared to rest of collaborators from the multiple collaborators (206) in other of two successive rounds.

**15.** A computer program comprising computer executable program code, when executed the program code controls a computer to perform the method (100) according to any one of claims 1-9.

FIG. 1

FIG. 2

FIG. 3

**EUROPEAN SEARCH REPORT**

Application Number

EP 23 21 6688

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHEN WANQI ET AL: "UCBFed: Using Reinforcement Learning Method to Tackle the Federated Optimization Problem", 9 June 2021 (2021-06-09), 20210609, PAGE(S) 99 - 105, XP047597932, [retrieved on 2021-06-09] | 1-7, 10-15 | INV.<br>G06N5/01<br>G06N20/00 |
| Y | * Abstract, Sections 2.2 and 3 * | 8,9 | |
| Y | ISIK-POLAT ECE ET AL: "Evaluation and Analysis of Different Aggregation and Hyperparameter Selection Methods for Federated Brain Tumor Segmentation", 15 July 2022 (2022-07-15), 20220715, PAGE(S) 405 - 419, XP047628604, [retrieved on 2022-07-15]<br>* Sections 2 and 4.2 * | 8,9 | |
| A | LARSSON HANNES HANNES LARSSON@ERICSSON COM ET AL: "Automated Collaborator Selection for Federated Learning with Multi-armed Bandit Agents", PROCEEDINGS OF THE 3RD WORKSHOP ON CYBER-SECURITY ARMS RACE, ACMPUB27, NEW YORK, NY, USA, 23 August 2021 (2021-08-23), pages 44-49, XP058769090, DOI: 10.1145/3472735.3473388 ISBN: 978-1-4503-8679-1<br>* Section 3 * | 1-7, 10-15 | |

TECHNICAL FIELDS SEARCHED (IPC)

G06N
G16H
G06T

-/--

The present search report has been drawn up for all claims

| Place of search | Date of completion of the search | Examiner |
|---|---|---|
| The Hague | 24 May 2024 | Targon, Valerio |

EPO FORM 1503 03.82 (P04C01)

page 1 of 2

Europäisches
Patentamt

European
Patent Office

Office européen
des brevets

# EUROPEAN SEARCH REPORT

**Application Number**

EP 23 21 6688

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MENG XUTAO ET AL: "An Optimization Method for Non-IID Federated Learning Based on Deep Reinforcement Learning", SENSORS, vol. 23, no. 22, 16 November 2023 (2023-11-16), page 9226, XP093164864, CH ISSN: 1424-8220, DOI: 10.3390/s23229226 * Sections 2-4 * | 1-7, 11-15 | |

-----

**TECHNICAL FIELDS SEARCHED (IPC)**

The present search report has been drawn up for all claims

| Place of search | Date of completion of the search | Examiner |
|---|---|---|
| The Hague | 24 May 2024 | Targon, Valerio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                       

& : member of the same patent family, corresponding document

EPO FORM 1503 03.82 (P04C01)